# EUROPEAN PATENT APPLICATION

(11) **EP 2 008 675 A1**
(43) Date of publication of application: **31.12.2008**
(21) Application number: 07730375.8
(22) Date of filing: 13.03.2007
(51) Int. Cl.: A61M 1/10, F04D 15/00, F04D 29/46

(54) **PUMP FOR BLOOD PERFUSION**

(30) Priority: 20.04.2006 ES 200601000
(71) Applicant: Merce Vives, Salvador, 46004 Valencia (ES)
(72) Inventor: Merce Vives, Salvador, 46004 Valencia (ES)
(74) Representative: Ungria Lopez, Javier
(86) International application number: PCT/ES2007/000134
(87) International publication number: WO 2007/122270

(57) **Abstract**

In blood perfusion during cardiac surgery or ventricular assistance, for treating cardiac insufficiency, use is currently made of a centrifugal pump that generates a linear flow in the circulatory system, counter to the pulsating flow generated by the heart. According to the invention, a pulsating linear flow is achieved for blood perfusion by providing, in the interior wall of the chamber or casing of the pump (3), an elastic lining (8) in the form of a bell-shaped membrane that inflates and deflates, producing variations in volume and changes in pressure that give rise to a pulsating effect in the flow at the outlet (6) from the pump (3). At the inlet (5) to the pump (3) there is a non-return valve formed by an annular thickening (11).

## Description

### OBJECT OF THE INVENTION

As stated in the title of this descriptive specification, the following invention relates to a pump for blood perfusion, with which notable advantages are achieved and which implies a technological advance in blood perfusion during cardiac surgery or ventricular assistance, for treating cardiac insufficiency.

It is an object of the invention to achieve a perfusion system that provides the advantages of linear or continuous flow generated by a centrifugal pump and also a pulsating flow, which is superior in the perfusion of tissues and organs, and which can also be synchronized with the biological pulse of the patient in the event that ventricular assistance is necessary.

### PRIOR ART OF THE INVENTION

Currently, both for blood perfusion during cardiac surgery and for treating cardiac insufficiency, two types of pump are used: roller pumps and centrifugal pumps. Both types of pump generate a linear or continuous flow and therefore a circulation of the blood at constant flow rather than the pulsating or physiological flow produced by the heart.

It has always been accepted that the ideal flow for perfusing the tissues is pulsating or physiological because the impact produced by each beat improves the perfusion of the narrowest arteries. The manufacturers of roller pumps and of centrifugal pumps have sought solutions for transforming linear flow into pulsating flow, without having achieved satisfactory results.

Invention Patent US 005458459 disclosed a centrifugal pump for pumping biological liquids such as blood, which includes a housing that defines a pumping chamber. The pumping chamber encloses a rotor (impellor) with blades having a distribution so as to form a rotary inductor which helps to reduce hemolysis, but which only generates non-physiological linear flow.

Invention Patent US 6183220 provides a blood pump capable of preventing in a substantially complete way the thrombi from adhering to the lower interior part of the jacket without reducing the anti-hemolytic characteristic of the blood. It comprises a pump jacket, a suction inlet arranged in the central lateral part of the jacket and a flow outlet arranged in the lower peripheral part, including a main rotor in order to create a centrifugal flow of blood supplied from the inlet. The rotor is provided with a mixer and stirring fins. In this case too, no synchronizable pulsating or physiological flow is generated.

### DESCRIPTION OF THE INVENTION

In general terms, the pump for blood perfusion, forming the object of this invention, permits a novel system of blood perfusion to be created with a linear and pulsating flow, contributing the advantages of the linear flow generated by a centrifugal pump (centrifugal pumps are less hemolytical) and the advantages of a pulsated flow (better perfusion of tissues and organs, and capacity for synchronization with the patient's pulse).

In short, a device is conceived which combines a centrifugal impulsion part, already known, with another pulsating impulsion part that is totally novel.

The pump used in accordance with the invention presents, like others of its type, a capsule or casing inside which the blood is received and, by means of a system of blades or vortex, the blood is made to rotate at high speed, generating a linear outlet flow by means of the centrifugal force. In order to be able to superimpose a pulsating flow on the linear flow provided by a centrifugal pump, coupled to the interior wall of that capsule or casing of the pump is an elastic lining or elastic bell-shaped membrane that is activated hydraulically or pneumatically (using a physiological serum or similar) causing it to inflate and deflate in order to produce variations in the interior volume of the capsule, generating the displacement of the same volume of blood which thus flows pulsating to the outlet of the centrifugal pump where it joins the linear flow. The interior lining or bell-shaped membrane is designed so that the elastic wall performs the function of a non-return valve at the inlet to the pump, so that the volume of blood is displaced solely in the direction of the outlet, coinciding with the linear flow of the centrifugal impulsion device.

The hydraulic transmission for the inflation and deflation of the chamber is carried out by means of a control console. This basically consists of a plunger which is displaced inside a jacket or cylinder, in the manner of a syringe. The plunger has alternating backwards and forwards movement with a frequency and travel that can be adjusted and made to synchronize with the biological pulse of the patient. The advantage of this system of perfusion with linear and pulsating flow compared to conventional linear flow pumps is that the blood circulation of the patient at all times has a basic flow for the perfusion of large vessels and a pulsating flow for boosting the perfusion of the small vessels.

The design of the elastic wall of the actual inflatable membrane, so that it can act as a non-return valve, is the preferred embodiment in order to thereby eliminate any other mechanical type of valve with more components and greater mechanical fatigue.

In order to facilitate an understanding of the characteristics of this invention and forming an integral part thereof, this specification is accompanied by two sheets of plans containing figures in which, on an illustrative rather than limiting basis, following have been represented:

### BRIEF DESCRIPTION OF THE DRAWINGS

**Figure 1****.-** Is a diagram of the linear pulsating flow and pressure generated according to the invention, in which the linear pressure, the pulsating pressure, the period and the pulse time can be regulated.
**Figure 2****.-** Is a schematic view in cross-section of a pump for blood perfusion, forming the object of the invention.

### DESCRIPTION OF THE PREFERRED FORM OF EMBODIMENT

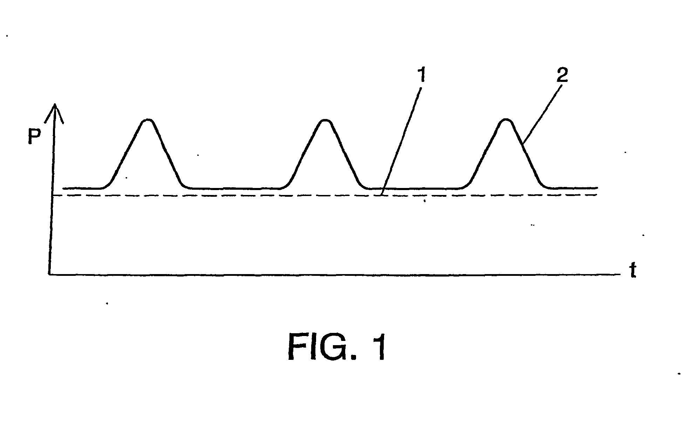

Making reference to the numbering adopted in the figures and especially in relation to figure 1, we can see that a centrifugal pump used for this purpose of perfusion receives the blood and, by centrifugal force, generates a linear outlet flow, as represented by the line of dashes 1 in the tP or tF diagram (time in the abscissa and the pressure P or flow F in the ordinate). With the system of the invention, the pulsating flow is superimposed on the linear flow, generating a pulsating wave 2 of adjustable frequency and amplitude by means of electronic control equipment linked to the hydraulic transmission equipment which causes the inflating and deflating of the elastic lining inside the pump. The values of pressure and flow, of both the linear and the pulsating component, thus manage to be varied, along with the period and time of the pulsating effect.

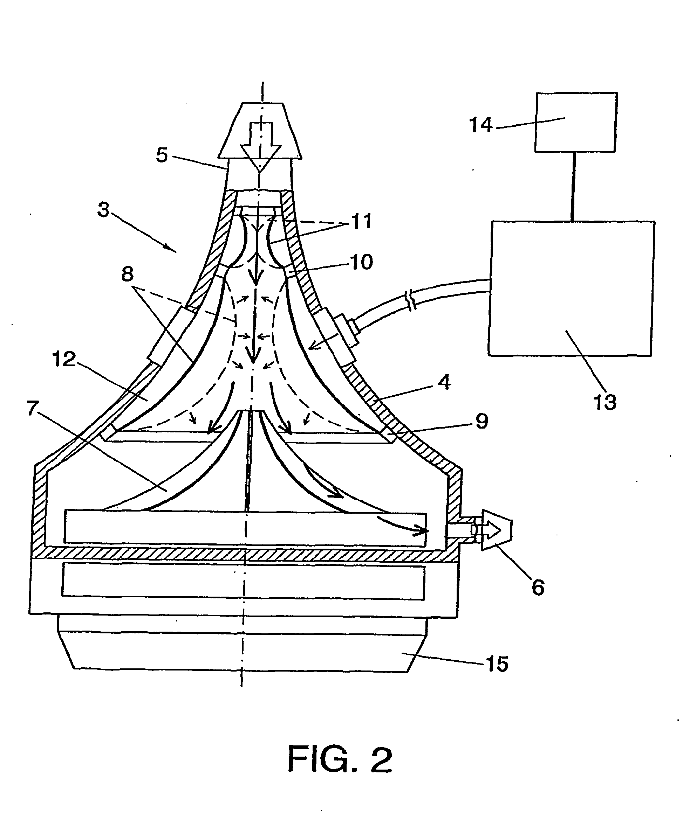

Figure 2 shows an example of embodiment in which the pump is referenced in general with the number 3 forming a capsule or casing 4 with an inlet 5 and an outlet 6, where inside the rotor 7 for blood impulsion rotates, suctioning the blood through the inlet 5 and sends a continuous flow through the outlet 6.

The reference 8 designates the internal elastic lining of the walls of the casing 4, in form of a bell-shaped membrane, which in this example of embodiment, has a ring 9 in its lower edge as a limiting element of the elasticity in that annular zone which is integral with and sealed to the walls of the capsule or casing 4. The upper edge also has another similar ring 10 and is continued upwards by an annular thickening of a non-return valve, referenced by 11, which is coaxial with the inlet 5 and does not block off the axial throat in the rest position. Only when the console 13 acts to inflate the membrane of the elastic lining 8 is it slightly and elastically deformed in order to close the entrance of the blood flow, acting as a non-return valve when the fluid tries to circulate in the opposite direction due to the effect of the pressure created by the pumping equipment which inflates and deflates the chamber 12 formed between the casing 4 and the membrane of the elastic lining 8.

As the fluid (preferably physiological serum or similar as we have said earlier) is made to arrive at this chamber 12, the internal volume is reduced and the blood pressure is increased in a manner that is pulsating and which is seen at the outlet 6.

Shown with dashed lines is the deformation of the bell-shaped membrane of the elastic lining 8 from its inactive position marked with a solid line.

The inflating and deflating of the chamber 12 is preferably achieved with the alternating forwards and backwards movements of the piston of a cylinder, the frequency and travel being controlled in line with the biological pulse of the patient via a console or equipment for action and control as schematically referenced with number 13 and a governing PC 14.

The reference 15 designates an external magnetic transmission plate in order to achieve the rotation of the impulsion rotor 7.

## Claims

1. **PUMP FOR BLOOD PERFUSION,** for generating the circulation of blood in the circulatory system during cardiac surgery or in ventricular assistance for treating cardiac insufficiency, **characterized in that** it provokes a pulsating linear flow when superimposed on the linear flow produced by the rotation of the impulsion rotor of the pump sending by means of centrifugal or vortex force a certain flow of blood which arrives at the inlet of the pump towards the outlet thereof; a pulsating flow generating a pulsating wave with adjustable frequency and amplitude that can be synchronized to the biological pulse of the patient, by introducing into the interior wall of the capsule or casing (4) of the pump (1) an elastic lining (8) which, when hydraulically or pneumatically activated, is inflated and deflated producing intermittent variations of volume in the interior of the capsule or casing (4) generating the displacement of a constant volume of blood which flows in a pulsated manner towards the outlet (6) due to the existence in the inlet (5) of a non-return valve (11).

2. **PUMP FOR BLOOD PERFUSION,** according to claim 1, **characterized in that** the elastic lining (8) acts in the inlet mouth as a non-return valve (11) formed by an annular thickening which manages to block the axial throat, being elastically opened in the direction of entrance of the flow and squeezing its walls when pressure is applied in the opposite direction in order to block the throat when a console (13) is actuated and the membrane or elastic lining (8) is inflated.

3. **PUMP FOR BLOOD PERFUSION,** according to the above claims, **characterized in that** the hydraulic or pneumatic activation produced by the alternative inflating and deflating of the elastic lining (8) is achieved by means of a control console (13) which essentially consists of a plunger that is displaced inside a cylinder with alternating forwards and backwards movement with adjustable frequency and travel that can be synchronized to the biological pulse of the patient, being connected to an inlet mouth which traverses the wall of the capsule or casing (4) of the pump and governed by the console (13) connected to a PC (14).

4. **PUMP FOR BLOOD PERFUSION,** according to the above claims, **characterized in that** the elastic lining (8) and the elastic thickening of the non-return valve (11) are connected so that the pressure exerted by the console (13) inside the elastic lining (8) is the same as that which acts in the non-return valve (11).
